# EUROPEAN PATENT SPECIFICATION

(11) **EP 3 993 747 B1**
(45) Date of publication and mention of the grant of the patent: **02.04.2025**
(21) Application number: 20736442.3
(22) Date of filing: 26.06.2020
(51) Int. Cl.: A61F 13/00, A61F 13/02

(54) **CUSTOMIZABLE DRESSINGS FOR NEGATIVE-PRESSURE TREATMENT OF LARGE AREAS**
ANPASSBARE VERBÄNDE ZUR UNTERDRUCKBEHANDLUNG GROSSER AREALE
PANSEMENTS PERSONNALISABLES POUR LE TRAITEMENT PAR PRESSION NÉGATIVE DE GRANDES ZONES

(30) Priority: 03.07.2019 US 201962870448 P
(43) Date of publication of application: 11.05.2022
(73) Proprietor: Solventum Intellectual Properties Company, Maplewood, MN 55144 (US)
(72) Inventor: ROBINSON, Timothy Mark, San Antonio, Texas 78265 (US); LOCKE, Christopher Brian, San Antonio, Texas 78265 (US)
(74) Representative: Simmons & Simmons
(86) International application number: PCT/IB2020/056072
(87) International publication number: WO 2021/001737

(56) References cited:
- WO-A1-2018/226650
- US-A1- 2010 087 767
- US-A1- 2018 353 342

## Description

### TECHNICAL FIELD

The invention set forth in the appended claims relates generally to tissue treatment systems and more particularly, but without limitation, to dressings for tissue treatment

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

There is also widespread acceptance that cleansing a tissue site can be highly beneficial for new tissue growth. For example, a wound or a cavity can be washed out with a liquid solution for therapeutic purposes. These practices are commonly referred to as "irrigation" and "lavage" respectively. "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed. WO 2018/226650 A1 concerns systems, apparatus and methods for negative-pressure treatment with reduced tissue in-growth. US 2018/353342 A1 concerns a peel and place dressing for negative-pressure therapy.

While the clinical benefits of negative-pressure therapy and/or instillation therapy are widely known, improvements to therapy systems, components, and processes may benefit healthcare providers and patients.

### BRIEF SUMMARY

The invention provides a dressing for treating a tissue site with negative pressure, as set forth in the appended claims. Illustrative embodiments are also provided to enable a person skilled in the art to make and use the claimed subject matter.

The dressing is a composite of dressing layers, including a perforated polymer film, a manifold, and an adhesive drape. The polymer film may be a polyethylene, polyurethane, or ethyl methyl acrylate (EMA) in some embodiments. The manifold may comprise or consist essentially of open-cell foam in some examples. The thickness of the manifold may vary for different types of tissue or fluid. For example, a foam manifold layer may be relatively thin and hydrophobic to reduce the fluid hold capacity of the dressing. The foam may also be thin to reduce the dressing profile and increase flexibility, which can enable it to conform to wound beds and other tissue sites under negative pressure. In other examples, a greater thickness may be advantageous for more viscous fluid or larger areas. The manifold may be adhered to the polymer film in some embodiments. Suitable bonds between the manifold and the polymer film may include pressure-sensitive adhesive (reactive and non-reactive types); hot melt adhesive (spray applied or deployed as a film, woven, or non-woven); hot press lamination; or flame lamination. The polymer film may also be co-extruded with a bonding layer in-situ, which may be formed from a hot melt adhesive, for example. The dressing may have an exposed perimeter, and the dressing may be cut to a desired size before applying the dressing to a tissue. Drape strips or other adhesive strips may be used to seal edges of the dressing and fix the dressing to a patient's skin.

Some dressings may also include a layer of low-tack adhesive, silicone, or other soft polymer layer having perforations. The perforation pattern of the polymer film can be aligned with the perforation pattern of at least a central area of the silicone. In some embodiments, the silicone may additionally include a pattern-coated acrylic, which can further facilitate fixation. For example, an acrylic adhesive can be applied about a peripheral area of the structure to increase bond strength in regions which are likely to be skin rather than a wound area.

In some embodiments, the second layer may have a perimeter that is exposed between the first layer and the cover, which can allow the dressing to be customized for size and shape. The manifold may be configured to maintain at least 80% of an applied negative pressure through the length.

In more particular examples, the perforations of the polymer film may comprise a plurality of slots or slits. The perforations may be elastic and configured to respond to a pressure gradient across the perforations. Some embodiments may further comprise a dressing interface configured to be fluidly coupled to the manifold through the cover. The dressing interface may be disposed at least 6 centimeters from an edge of the manifold.

The cover may comprise a non-porous film, and the second layer (manifold) may be adhered to the non-porous film. The first layer (fluid-control layer) may be adhered to the manifold opposite the non-porous film, so that the cover, the manifold, and the fluid-control layer are arranged in a stack with the manifold between the cover and the fluid-control layer. The manifold may have a perimeter that is exposed between the cover and the fluid-control layer. The manifold can be configured to maintain at least 80% of a negative pressure through a distance of at least 6 centimeters. In some examples, the dressing may further comprise a fluid port coupled to the cover and fluidly coupled to the manifold through the cover. An attachment device may be configured to seal the perimeter.

A method for treating a tissue site with negative pressure is also described herein, wherein some example embodiments include applying a dressing to the tissue site, wherein the dressing comprises a manifold. A fluid conductor may be fluidly coupled to the manifold and to a negative-pressure source. Negative pressure from the negative-pressure source may be applied to the manifold through the fluid conductor, and at least 80% of the negative pressure in the manifold can be maintained for a distance of at least 6 centimeters from the fluid conductor. In some examples, the manifold may have a thickness in a range of about 7 millimeters to about 9 millimeters. A thickness of about 8 millimeters may be particularly advantageous for some applications.

Other objectives, advantages, and a preferred mode of making and using the claimed subject matter may be understood best by reference to the accompanying drawings in conjunction with the following detailed description of illustrative embodiments.

### BRIEF DESCRIPTION OF THE DRAWINGS

Figure 1 is a functional block diagram of an example embodiment of a therapy system that can provide negative-pressure treatment and instillation treatment in accordance with this specification;
Figure 2 is an assembly view of an example of a dressing that can be associated with some embodiments of the therapy system of Figure 1;
Figure 3 is a schematic view of an example layer that can be associated with some embodiments of the dressing of Figure 2;
Figure 4 is a side view of an example of the dressing of Figure 2;
Figure 5 is an assembly view of another example of a dressing that can be associated with some embodiments of the therapy system of Figure 1;
Figure 6 is a schematic view of an example layer that can be associated with some embodiments of the dressing of Figure 5;
Figure 7 is a schematic view of the example layer of Figure 6 overlaid on the example layer of Figure 3;
Figure 8 is an assembly view of another example of a dressing that may be associated with some embodiments of the therapy system of Figure 1;
Figure 9 is a schematic diagram of an example of the therapy system of Figure 1 applied to a tissue site; and
Figure 10 is a line chart that illustrates how the thickness of a manifold can affect the manifolding performance in the presence of thick exudate.

### DESCRIPTION OF EXAMPLE EMBODIMENTS

The following description of example embodiments provides information that enables a person skilled in the art to make and use the subject matter set forth in the appended claims, but it may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

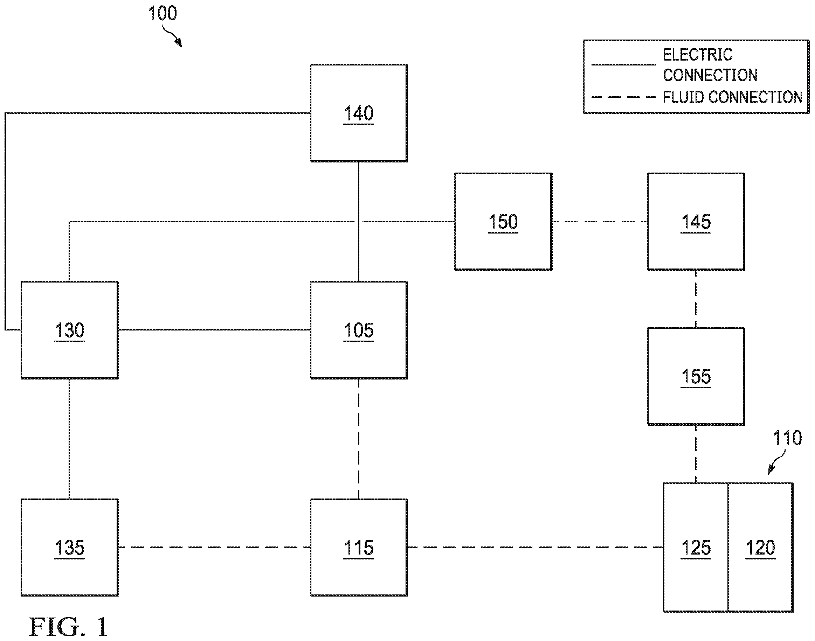

Figure 1 is a simplified functional block diagram of an example embodiment of a therapy system 100 that can provide negative-pressure therapy with instillation of topical treatment solutions to a tissue site in accordance with this specification.

The term "tissue site" in this context broadly refers to a wound, defect, or other treatment target located on or within tissue, including but not limited to, a surface wound, bone tissue, adipose tissue, muscle tissue, neural tissue, dermal tissue, vascular tissue, connective tissue, cartilage, tendons, or ligaments. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted. A surface wound, as used herein, is a wound on a body that is exposed to the external environment, such as an injury or damage to the epidermis, dermis, and/or subcutaneous layers. Surface wounds may include ulcers or closed incisions, for example. A surface wound, as used herein, does not include wounds within an intra-abdominal cavity. A wound may include chronic, acute, traumatic, subacute, and dehisced wounds, partial-thickness burns, ulcers (such as diabetic, pressure, or venous insufficiency ulcers), flaps, and grafts, for example.

The therapy system 100 may include a source or supply of negative pressure, such as a negative-pressure source 105, and one or more distribution components. A distribution component is preferably detachable and may be disposable, reusable, or recyclable. A dressing, such as a dressing 110, and a fluid container, such as a container 115, are examples of distribution components that may be associated with some examples of the therapy system 100. As illustrated in the example of Figure 1, the dressing 110 may comprise or consist essentially of a tissue interface 120, a cover 125, or both in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface may facilitate coupling a fluid conductor to the dressing 110. For example, such a dressing interface may be a SENSAT.R.A.C.^{™} Pad available from Kinetic Concepts, Inc. of San Antonio, Texas.

The therapy system 100 may also include a regulator or controller, such as a controller 130. Additionally, the therapy system 100 may include sensors to measure operating parameters and provide feedback signals to the controller 130 indicative of the operating parameters. As illustrated in Figure 1, for example, the therapy system 100 may include a first sensor 135 and a second sensor 140 coupled to the controller 130.

The therapy system 100 may also include a source of instillation solution. For example, a solution source 145 may be fluidly coupled to the dressing 110, as illustrated in the example embodiment of Figure 1. The solution source 145 may be fluidly coupled to a positive-pressure source, such as a positive-pressure source 150, a negative-pressure source such as the negative-pressure source 105, or both in some embodiments. A regulator, such as an instillation regulator 155, may also be fluidly coupled to the solution source 145 and the dressing 110 to ensure proper dosage of instillation solution (e.g. saline) to a tissue site. For example, the instillation regulator 155 may comprise a piston that can be pneumatically actuated by the negative-pressure source 105 to draw instillation solution from the solution source during a negative-pressure interval and to instill the solution to a dressing during a venting interval. Additionally or alternatively, the controller 130 may be coupled to the negative-pressure source 105, the positive-pressure source 150, or both, to control dosage of instillation solution to a tissue site. In some embodiments, the instillation regulator 155 may also be fluidly coupled to the negative-pressure source 105 through the dressing 110, as illustrated in the example of Figure 1.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 105 may be combined with the controller 130, the solution source 145, and other components into a therapy unit.

In general, components of the therapy system 100 may be coupled directly or indirectly. For example, the negative-pressure source 105 may be directly coupled to the container 115 and may be indirectly coupled to the dressing 110 through the container 115. Coupling may include fluid, mechanical, thermal, electrical, or chemical coupling (such as a chemical bond), or some combination of coupling in some contexts. For example, the negative-pressure source 105 may be electrically coupled to the controller 130 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 105, may be a reservoir of air at a negative pressure or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 105 may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-6.7 kPa) and -300 mm Hg (-39.9 kPa).

The container 115 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 130, may be a microprocessor or computer programmed to operate one or more components of the therapy system 100, such as the negative-pressure source 105. In some embodiments, for example, the controller 130 may be a microcontroller, which generally comprises an integrated circuit containing a processor core and a memory programmed to directly or indirectly control one or more operating parameters of the therapy system 100. Operating parameters may include the power applied to the negative-pressure source 105, the pressure generated by the negative-pressure source 105, or the pressure distributed to the tissue interface 120, for example. The controller 130 is also preferably configured to receive one or more input signals, such as a feedback signal, and programmed to modify one or more operating parameters based on the input signals.

Sensors, such as the first sensor 135 and the second sensor 140, are generally known in the art as any apparatus operable to detect or measure a physical phenomenon or property, and generally provide a signal indicative of the phenomenon or property that is detected or measured. For example, the first sensor 135 and the second sensor 140 may be configured to measure one or more operating parameters of the therapy system 100. In some embodiments, the first sensor 135 may be a transducer configured to measure pressure in a pneumatic pathway and convert the measurement to a signal indicative of the pressure measured. In some embodiments, for example, the first sensor 135 may be a piezo-resistive strain gauge. The second sensor 140 may optionally measure operating parameters of the negative-pressure source 105, such as a voltage or current, in some embodiments. Preferably, the signals from the first sensor 135 and the second sensor 140 are suitable as an input signal to the controller 130, but some signal conditioning may be appropriate in some embodiments. For example, the signal may need to be filtered or amplified before it can be processed by the controller 130. Typically, the signal is an electrical signal, but may be represented in other forms, such as an optical signal.

The tissue interface 120 can be generally adapted to partially or fully contact a tissue site. The tissue interface 120 may take many forms, and may have many sizes, shapes, or thicknesses, depending on a variety of factors, such as the type of treatment being implemented or the nature and size of a tissue site. For example, the size and shape of the tissue interface 120 may be adapted to the contours of deep and irregular shaped tissue sites. Any or all of the surfaces of the tissue interface 120 may have an uneven, coarse, or jagged profile.

In some embodiments, the tissue interface 120 may comprise or consist essentially of a manifold. A manifold in this context may comprise or consist essentially of a means for collecting or distributing fluid across the tissue interface 120 under pressure. For example, a manifold may be adapted to receive negative pressure from a source and distribute negative pressure through multiple apertures across the tissue interface 120, which may have the effect of collecting fluid from across a tissue site and drawing the fluid toward the source. In some embodiments, the fluid path may be reversed or a secondary fluid path may be provided to facilitate delivering fluid, such as fluid from a source of instillation solution, across a tissue site.

In some embodiments, the cover 125 may provide a bacterial barrier and protection from physical trauma. The cover 125 may also be constructed from a material that can reduce evaporative losses and provide a fluid seal between two components or two environments, such as between a therapeutic environment and a local external environment. The cover 125 may comprise or consist of, for example, an elastomeric film or membrane that can provide a seal adequate to maintain a negative pressure at a tissue site for a given negative-pressure source. The cover 125 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties.

In some example embodiments, the cover 125 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 125 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polyamide copolymers. Such materials are commercially available as, for example, Tegaderm^{®} drape, commercially available from 3M Company, Minneapolis Minnesota; polyurethane (PU) drape; polyether block polyamide copolymer (PEBAX), for example; and INSPIRE 2301 and INSPIRE 2327 polyurethane films, commercially available from Expopack Advanced Coatings, Wrexham, United Kingdom. In some embodiments, the cover 125 may comprise INSPIRE 2301 having an MVTR (upright cup technique) of 2600 g/m²/24 hours and a thickness of about 30 microns.

An attachment device may be used to attach the cover 125 to an attachment surface, such as undamaged epidermis, a gasket, or another cover. The attachment device may take many forms. For example, an attachment device may be a medically-acceptable, pressure-sensitive adhesive configured to bond the cover 125 to epidermis around a tissue site. In some embodiments, for example, some or all of the cover 125 may be coated with an adhesive, such as an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The solution source 145 may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

In operation, the tissue interface 120 may be placed within, over, on, or otherwise proximate to a tissue site. If the tissue site is a wound, for example, the tissue interface 120 may partially or completely fill the wound, or it may be placed over the wound. The cover 125 may be placed over the tissue interface 120 and sealed to an attachment surface near a tissue site. For example, the cover 125 may be sealed to undamaged epidermis peripheral to a tissue site. Thus, the dressing 110 can provide a sealed therapeutic environment proximate to a tissue site, substantially isolated from the external environment, and the negative-pressure source 105 can reduce pressure in the sealed therapeutic environment.

The fluid mechanics of using a negative-pressure source to reduce pressure in another component or location, such as within a sealed therapeutic environment, can be mathematically complex. However, the basic principles of fluid mechanics applicable to negative-pressure therapy and instillation are generally well-known to those skilled in the art, and the process of reducing pressure may be described illustratively herein as "delivering," "distributing," or "generating" negative pressure, for example.

In general, exudate and other fluid flow toward lower pressure along a fluid path. Thus, the term "downstream" typically implies something in a fluid path relatively closer to a source of negative pressure or further away from a source of positive pressure. Conversely, the term "upstream" implies something relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications, such as by substituting a positive-pressure source for a negative-pressure source, and this descriptive convention should not be construed as a limiting convention.

Negative pressure applied across the tissue site through the tissue interface 120 in the sealed therapeutic environment can induce macro-strain and micro-strain in the tissue site. Negative pressure can also remove exudate and other fluid from a tissue site, which can be collected in container 115.

In some embodiments, the controller 130 may receive and process data from one or more sensors, such as the first sensor 135. The controller 130 may also control the operation of one or more components of the therapy system 100 to manage the pressure delivered to the tissue interface 120. In some embodiments, controller 130 may include an input for receiving a desired target pressure and may be programmed for processing data relating to the setting and inputting of the target pressure to be applied to the tissue interface 120. In some example embodiments, the target pressure may be a fixed pressure value set by an operator as the target negative pressure desired for therapy at a tissue site and then provided as input to the controller 130. The target pressure may vary from tissue site to tissue site based on the type of tissue forming a tissue site, the type of injury or wound (if any), the medical condition of the patient, and the preference of the attending physician. After selecting a desired target pressure, the controller 130 can operate the negative-pressure source 105 in one or more control modes based on the target pressure and may receive feedback from one or more sensors to maintain the target pressure at the tissue interface 120.

In some embodiments, the controller 130 may have a continuous pressure mode, in which the negative-pressure source 105 is operated to provide a constant target negative pressure for the duration of treatment or until manually deactivated. Additionally or alternatively, the controller may have an intermittent pressure mode. For example, the controller 130 can operate the negative-pressure source 105 to cycle between a target pressure and atmospheric pressure. For example, the target pressure may be set at a value of 135 mmHg for a specified period of time (e.g., 5 min), followed by a specified period of time (e.g., 2 min) of deactivation. The cycle can be repeated by activating the negative-pressure source 105, which can form a square wave pattern between the target pressure and atmospheric pressure.

In some example embodiments, the increase in negative-pressure from ambient pressure to the target pressure may not be instantaneous. For example, the negative-pressure source 105 and the dressing 110 may have an initial rise time. The initial rise time may vary depending on the type of dressing and therapy equipment being used. For example, some therapy systems may increase negative pressure at a rate of about 20-30 mmHg/second, and other therapy systems may increase negative pressure at a rate of about 5-10 mmHg/second. If the therapy system 100 is operating in an intermittent mode, the repeating rise time may be a value substantially equal to the initial rise time.

In some example dynamic pressure control modes, the target pressure can vary with time. For example, the target pressure may vary in the form of a triangular waveform, varying between a negative pressure of 50 and 135 mmHg with a rise rate of negative pressure set at a rate of 25 mmHg/min. and a descent rate set at 25 mmHg/min. In other embodiments of the therapy system 100, the triangular waveform may vary between negative pressure of 25 and 135 mmHg with a rise rate of about 30 mmHg/min and a descent rate set at about 30 mmHg/min.

In some embodiments, the controller 130 may control or determine a variable target pressure in a dynamic pressure mode, and the variable target pressure may vary between a maximum and minimum pressure value that may be set as an input prescribed by an operator as the range of desired negative pressure. The variable target pressure may also be processed and controlled by the controller 130, which can vary the target pressure according to a predetermined waveform, such as a triangular waveform, a sine waveform, or a saw-tooth waveform. In some embodiments, the waveform may be set by an operator as the predetermined or time-varying negative pressure desired for therapy.

In some embodiments, the controller 130 may receive and process data, such as data related to instillation solution provided to the tissue interface 120. Such data may include the type of instillation solution prescribed by a clinician, the volume of fluid or solution to be instilled to a tissue site ("fill volume"), and the amount of time prescribed for leaving solution at a tissue site ("dwell time") before applying a negative pressure to the tissue site. The fill volume may be, for example, between 10 and 500 mL, and the dwell time may be between one second to 30 minutes. The controller 130 may also control the operation of one or more components of the therapy system 100 to instill solution. For example, the controller 130 may manage fluid distributed from the solution source 145 to the tissue interface 120. In some embodiments, fluid may be instilled to a tissue site by applying a negative pressure from the negative-pressure source 105 to reduce the pressure at the tissue site, drawing solution into the tissue interface 120. In some embodiments, solution may be instilled to a tissue site by applying a positive pressure from the positive-pressure source 160 to move solution from the solution source 145 to the tissue interface 120. Additionally or alternatively, the solution source 145 may be elevated to a height sufficient to allow gravity to move solution into the tissue interface 120.

The controller 130 may also control the fluid dynamics of instillation by providing a continuous flow of solution or an intermittent flow of solution. Negative pressure may be applied to provide either continuous flow or intermittent flow of solution. The application of negative pressure may be implemented to provide a continuous pressure mode of operation to achieve a continuous flow rate of instillation solution through the tissue interface 120, or it may be implemented to provide a dynamic pressure mode of operation to vary the flow rate of instillation solution through the tissue interface 120. Alternatively, the application of negative pressure may be implemented to provide an intermittent mode of operation to allow instillation solution to dwell at the tissue interface 120. In an intermittent mode, a specific fill volume and dwell time may be provided depending, for example, on the type of tissue site being treated and the type of dressing being utilized. After or during instillation of solution, negative-pressure treatment may be applied. The controller 130 may be utilized to select a mode of operation and the duration of the negative pressure treatment before commencing another instillation cycle.

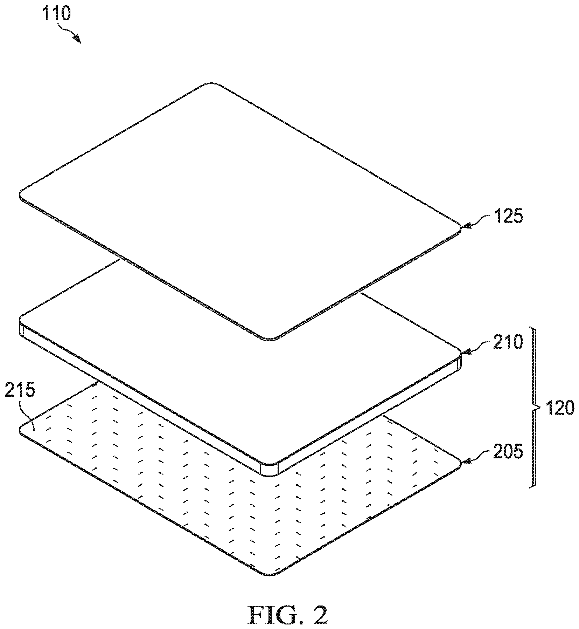

Figure 2 is an assembly view of an example of the dressing 110 of Figure 1, illustrating additional details that may be associated with some embodiments in which the tissue interface 120 comprises more than one layer. In the example of Figure 2, the tissue interface comprises a first layer 205 and a second layer 210. In some embodiments, the first layer 205 may be disposed adjacent to the second layer 210. For example, the first layer 205 and the second layer 210 may be stacked so that the first layer 205 is in contact with the second layer 210. The first layer 205 may also be heat-bonded or adhered to the second layer 210 in some embodiments. In some embodiments, the first layer 205 optionally includes a low-tack adhesive, which can be configured to hold the tissue interface 120 in place while the cover 125 is applied. The low-tack adhesive may be continuously coated on the first layer 205 or applied in a pattern.

The first layer 205 may comprise or consist essentially of a means for controlling or managing fluid flow. In some embodiments, the first layer 205 may be a fluid control layer comprising or consisting essentially of a liquid-impermeable, elastomeric material. For example, the first layer 205 may comprise or consist essentially of a polymer film, such as a polyurethane film. In some embodiments, the first layer 205 may comprise or consist essentially of the same material as the cover 125. The first layer 205 may also have a smooth or matte surface texture in some embodiments. A glossy or shiny finish finer or equal to a grade B3 according to the SPI (Society of the Plastics Industry) standards may be particularly advantageous for some applications. In some embodiments, variations in surface height may be limited to acceptable tolerances. For example, the surface of the first layer 205 may have a substantially flat surface, with height variations limited to 0.2 millimeters over a centimeter.

In some embodiments, the first layer 205 may be hydrophobic. The hydrophobicity of the first layer 205 may vary, but may have a contact angle with water of at least ninety degrees in some embodiments. In some embodiments the first layer 205 may have a contact angle with water of no more than 150 degrees. For example, in some embodiments, the contact angle of the first layer 205 may be in a range of at least 90 degrees to about 120 degrees, or in a range of at least 120 degrees to 150 degrees. Water contact angles can be measured using any standard apparatus. Although manual goniometers can be used to visually approximate contact angles, contact angle measuring instruments can often include an integrated system involving a level stage, liquid dropper such as a syringe, camera, and software designed to calculate contact angles more accurately and precisely, among other things. Nonlimiting examples of such integrated systems may include the FTÅ125, FTÅ200, FTÅ2000, and FTÅ4000 systems, all commercially available from First Ten Angstroms, Inc., of Portsmouth, VA, and the DTA25, DTA30, and DTA100 systems, all commercially available from Kruss GmbH of Hamburg, Germany. Unless otherwise specified, water contact angles herein are measured using deionized and distilled water on a level sample surface for a sessile drop added from a height of no more than 5 cm in air at 20-25°C and 20-50% relative humidity. Contact angles herein represent averages of 5-9 measured values, discarding both the highest and lowest measured values. The hydrophobicity of the first layer 205 may be further enhanced with a hydrophobic coating of other materials, such as silicones and fluorocarbons, either as coated from a liquid, or plasma coated.

The first layer 205 may also be suitable for welding to other layers, including the second layer 210. For example, the first layer 205 may be adapted for welding to polyurethane foams using heat, radio frequency (RF) welding, or other methods to generate heat such as ultrasonic welding. RF welding may be particularly suitable for more polar materials, such as polyurethane, polyamides, polyesters and acrylates. Sacrificial polar interfaces may be used to facilitate RF welding of less polar film materials, such as polyethylene.

The area density of the first layer 205 may vary according to a prescribed therapy or application. In some embodiments, an area density of less than 40 grams per square meter may be suitable, and an area density of about 20-30 grams per square meter may be particularly advantageous for some applications.

In some embodiments, for example, the first layer 205 may comprise or consist essentially of a hydrophobic polymer, such as a polyethylene film. The simple and inert structure of polyethylene can provide a surface that interacts little, if any, with biological tissues and fluids, providing a surface that may encourage the free flow of liquids and low adherence, which can be particularly advantageous for many applications. Other suitable polymeric films include polyurethanes, acrylics, polyolefin (such as cyclic olefin copolymers), polyacetates, polyamides, polyesters, copolyesters, PEBAX block copolymers, thermoplastic elastomers, thermoplastic vulcanizates, polyethers, polyvinyl alcohols, polypropylene, polymethylpentene, polycarbonate, styreneics, silicones, fluoropolymers, and acetates. A thickness between 20 microns and 100 microns may be suitable for many applications. Films may be clear, colored, or printed. More polar films suitable for laminating to a polyethylene film include polyamide, co-polyesters, ionomers, and acrylics. To aid in the bond between a polyethylene and polar film, tie layers may be used, such as ethylene vinyl acetate, or modified polyurethanes. An ethyl methyl acrylate (EMA) film may also have suitable hydrophobic and welding properties for some configurations.

The first layer 205 may have one or more passages, which can be distributed uniformly or randomly across the first layer 205. The passages may be bi-directional and pressure-responsive. For example, each of the passages generally may comprise or consist essentially of an elastic passage that is normally unstrained to substantially reduce liquid flow, and can expand or open in response to a pressure gradient. As illustrated in the example of Figure 2, the passages may comprise or consist essentially of perforations 215 in the first layer 205. Perforations 215 may be formed by removing material from the first layer 205. For example, perforations 215 may be formed by cutting through the first layer 205. In the absence of a pressure gradient across the perforations 215, the perforations 215 may be sufficiently small to form a seal or fluid restriction, which can substantially reduce or prevent liquid flow. Additionally, or alternatively, one or more of the passages may be or may function as an elastomeric valve that is normally closed when unstrained to substantially prevent liquid flow, and can open in response to a pressure gradient. In some examples, the passages may comprise or consist essentially of fenestrations in the first layer 205. Generally, fenestrations are a species of perforation, and may also be formed by removing material from the first layer 205. The amount of material removed and the resulting dimensions of the fenestrations may be up to an order of magnitude less than perforations.

In some embodiments, the perforations 215 may be formed as slots (or fenestrations formed as slits) in the first layer 205. In some examples, the perforations 215 may comprise or consist of linear slots having a length less than 4 millimeters and a width less than 1 millimeter. The length may be at least 2 millimeters, and the width may be at least 0.4 millimeters in some embodiments. A length of about 3 millimeters and a width of about 0.8 millimeters may be particularly suitable for many applications, and a tolerance of about 0.1 millimeter may also be acceptable. Such dimensions and tolerances may be achieved with a laser cutter, for example. Slots of such configurations may function as imperfect elastomeric valves that can substantially reduce liquid flow in a normally closed or resting state. For example, such slots may form a flow restriction without being completely closed or sealed. The slots can expand or open wider in response to a pressure gradient to allow increased liquid flow.

The second layer 210 generally comprises or consists essentially of a manifold or a manifold layer, which can provide a means for collecting or distributing fluid across the tissue interface 120 under pressure. For example, the second layer 210 may be adapted to receive negative pressure from a source and distribute negative pressure through multiple apertures across the tissue interface 120, which may have the effect of collecting fluid from across a tissue site and drawing the fluid toward the source. In some embodiments, the fluid path may be reversed or a secondary fluid path may be provided to facilitate delivering fluid, such as from a source of instillation solution, across the tissue interface 120.

In some illustrative embodiments, the pathways of the second layer 210 may be interconnected to improve distribution or collection of fluids. In some illustrative embodiments, the second layer 210 may comprise or consist essentially of a porous material having interconnected fluid pathways. Examples of suitable porous material that comprise or can be adapted to form interconnected fluid pathways (e.g., channels) may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, the second layer 210 may additionally or alternatively comprise projections that form interconnected fluid pathways. For example, the second layer 210 may be molded to provide surface projections that define interconnected fluid pathways.

In some embodiments, the second layer 210 may comprise or consist essentially of a reticulated foam having pore sizes and free volume that may vary according to needs of a prescribed therapy. For example, a reticulated foam having a free volume of at least 90% may be suitable for many therapy applications, and a foam having an average pore size in a range of 400-600 microns may be particularly suitable for some types of therapy. The tensile strength of the second layer 210 may also vary according to needs of a prescribed therapy. For example, the tensile strength of a foam may be increased for instillation of topical treatment solutions. The 25% compression load deflection of the second layer 210 may be at least 0.35 pounds per square inch, and the 65% compression load deflection may be at least 0.43 pounds per square inch. In some embodiments, the tensile strength of the second layer 210 may be at least 10 pounds per square inch. The second layer 210 may have a tear strength of at least 2.5 pounds per inch. In some embodiments, the second layer 210 may be a foam comprised of polyols such as polyester or polyether, isocyanate such as toluene diisocyanate, and polymerization modifiers such as amines and tin compounds. In some examples, the second layer 210 may be a reticulated polyurethane foam such as used in GRANUFOAM^{™} dressing or V.A.C. VERAFLO^{™} dressing, both available from KCI of San Antonio, Texas.

Other suitable materials for the second layer 210 may include non-woven fabrics; three-dimensional (3D) polymeric structures, such as molded polymers, embossed and formed films, and fusion-bonded films, and mesh, for example.

In some examples, the second layer 210 may include a 3D textile. A 3D textile of polyester fibers may be particularly advantageous for some embodiments. For example, the second layer 210 may comprise or consist essentially of a three-dimensional weave of polyester fibers. In some embodiments, the fibers may be elastic in at least two dimensions. A puncture-resistant fabric of polyester and cotton fibers having a weight of about 650 grams per square meter and a thickness of about 1-2 millimeters may be particularly advantageous for some embodiments. Such a puncture-resistant fabric may have a warp tensile strength of about 330-340 kilograms and a weft tensile strength of about 270-280 kilograms in some embodiments. Another particularly suitable material may be a polyester spacer fabric having a weight of about 470 grams per square meter, which may have a thickness of about 4-5 millimeters in some embodiments. Such a spacer fabric may have a compression strength of about 20-25 kilopascals (at 40% compression). Additionally or alternatively, the second layer 210 may comprise or consist of a material having substantial linear stretch properties, such as a polyester spacer fabric having 2-way stretch and a weight of about 380 grams per square meter. A suitable spacer fabric may have a thickness of about 3-4 millimeters, and may have a warp and weft tensile strength of about 30-40 kilograms in some embodiments. The fabric may have a close-woven layer of polyester on one or more opposing faces in some examples.

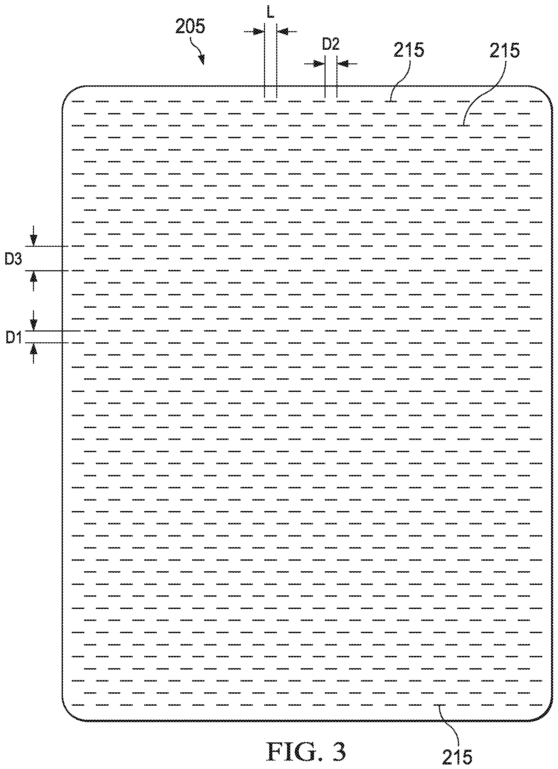

Figure 3 is a schematic view of another example of the first layer 205, illustrating additional details that may be associated with some embodiments. As illustrated in the example of Figure 3, the perforations 215 may each consist essentially of one or more linear slots having a length L. A length L of about 3 millimeters may be suitable for some examples. Figure 3 additionally illustrates an example of a uniform distribution pattern of the perforations 215. In Figure 3, the perforations 215 are substantially coextensive with the first layer 205, and are distributed across the first layer 205 in a grid of parallel rows and columns, in which the slots are also mutually parallel to each other. The rows may be spaced a distance D1, and the perforations 215 within each of the rows may be spaced a distance D2. For example, a distance D1 of about 3 millimeters on center and a distance D2 of about 3 millimeters on center may be suitable for some embodiments. The perforations 215 in adjacent rows may be aligned or offset. For example, adjacent rows may be offset, as illustrated in Figure 3, so that the perforations 215 are aligned in alternating rows separated by a distance D3. A distance D3 of about 6 millimeters may be suitable for some examples. The spacing of the perforations 215 may vary in some embodiments to increase the density of the perforations 215 according to therapeutic requirements.

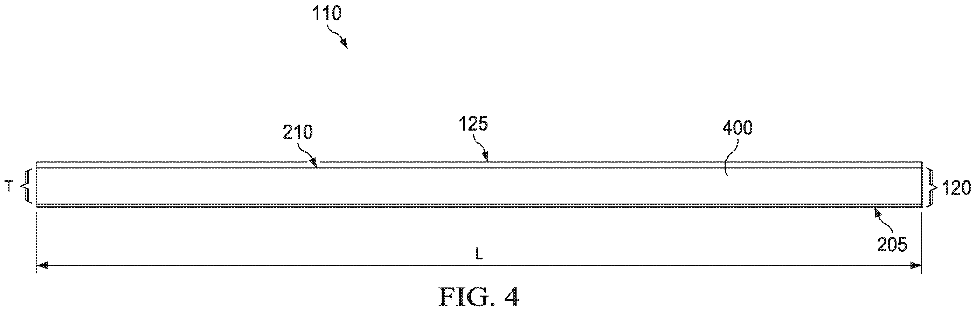

Figure 4 is a side view of an example of the dressing 110 of Figure 2 that may be associated with some embodiments of the therapy system of Figure 1. As shown in Figure 4, the tissue interface 120 has an exposed perimeter 400. More particularly, in the example of Figure 4, the cover 125, the first layer 205, and the second layer 210 each have an exposed perimeter, and there is no seam, weld, or seal along the exposed perimeter 400.

The second layer 210 generally has a first planar surface and a second planar surface opposite the first planar surface. The thickness T of the second layer 210 between the first planar surface and the second planar surface may also vary according to needs of a prescribed therapy. For example, the thickness T of the second layer 210 may be decreased to relieve stress on other layers and to reduce tension on peripheral tissue. The thickness of the second layer 210 can also affect the conformability and manifolding performance of the second layer 210. In some embodiments, a suitable foam may have a thickness T in a range of about 5 millimeters to 10 millimeters. In other examples, a suitable foam having a thickness T in a range of about 6 millimeters to about 12 millimeters may be suitable, and a thickness T of at least 8 millimeters may be advantageous. Fabrics, including suitable 3D textiles and spacer fabrics, may have a thickness T in a range of about 2 millimeters to about 8 millimeters. The second layer 210 also has a length L, which can vary according needs of a particular tissue site or prescribed therapy. A length L in a range of about 3 millimeters to about 30 millimeters may be suitable for some applications.

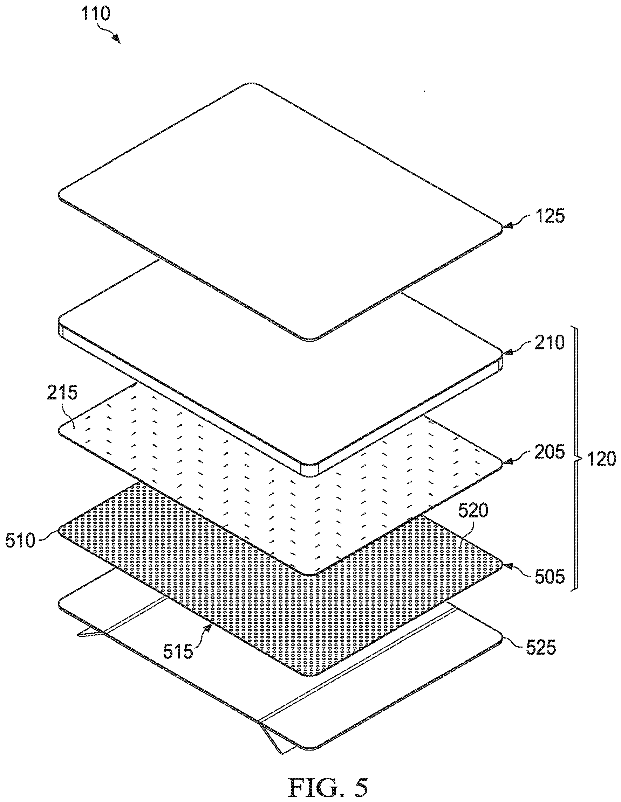

Figure 5 is an assembly view of another example of the dressing 110 of Figure 1, illustrating additional details that may be associated with some embodiments in which the tissue interface 120 may comprise additional layers. In the example of Figure 5, the tissue interface 120 comprises a third layer 505, in addition to the first layer 205 and the second layer 210. In some embodiments, the third layer 505 may be adjacent to the first layer 205 opposite the second layer 210. The third layer 505 may also be bonded to the first layer 205 in some embodiments.

The third layer 505 may comprise or consist essentially of a sealing layer formed from a soft, pliable material, such as a tacky gel, suitable for providing a fluid seal with a tissue site, and may have a substantially flat surface. For example, the third layer 505 may comprise, without limitation, a silicone gel, a soft silicone, hydrocolloid, hydrogel, polyurethane gel, polyolefin gel, hydrogenated styrenic copolymer gel, a foamed gel, a soft closed cell foam such as polyurethanes and polyolefins coated with an adhesive, polyurethane, polyolefin, or hydrogenated styrenic copolymers. The third layer 505 may include an adhesive surface on an underside and a patterned coating of acrylic on a top side. The patterned coating of acrylic may be applied about a peripheral area to allow higher bonding in regions that are likely to be in contact with skin rather than the wound area. In other embodiments, the third layer 505 may comprise a low-tack adhesive layer instead of silicone. In some embodiments, the third layer 505 may have a thickness between about 200 microns (µm) and about 1000 microns (µm). In some embodiments, the third layer 505 may have a hardness between about 5 Shore OO and about 80 Shore OO. Further, the third layer 505 may be comprised of hydrophobic or hydrophilic materials.

In some embodiments, the third layer 505 may be a hydrophobic-coated material. For example, the third layer 505 may be formed by coating a porous material, such as, for example, woven, nonwoven, molded, or extruded mesh with a hydrophobic material. The hydrophobic material for the coating may be a soft silicone, for example.

The third layer 505 may have corners 510 and edges 515. The third layer 505 may include apertures 520. The apertures 520 may be formed by cutting or by application of local RF or ultrasonic energy, for example, or by other suitable techniques for forming an opening. The apertures 520 may have a uniform distribution pattern, or may be randomly distributed on the third layer 505. The apertures 520 in the third layer 505 may have many shapes, including circles, squares, stars, ovals, polygons, slits, complex curves, rectilinear shapes, triangles, for example, or may have some combination of such shapes.

Each of the apertures 520 may have uniform or similar geometric properties. For example, in some embodiments, each of the apertures 520 may be circular apertures, having substantially the same diameter. In some embodiments, the diameter of each of the apertures 520 may be between about 1 millimeter and about 50 millimeters. In other embodiments, the diameter of each of the apertures 520 may be between about 1 millimeter and about 20 millimeters.

In other embodiments, geometric properties of the apertures 520 may vary. For example, the diameter of the apertures 520 may vary depending on the position of the apertures 520 in the third layer 505. The apertures 520 may be spaced substantially equidistant over the third layer 505. Alternatively, the spacing of the apertures 520 may be irregular.

As illustrated in the example of Figure 5, some embodiments of the dressing 110 may include a release liner 525 to protect the third layer 505 prior to use. The release liner 525 may also provide stiffness to facilitate handling and applying the dressing 110. The release liner 525 may be, for example, a casting paper, a film, or polyethylene. Further, in some embodiments, the release liner 525 may be a polyester material such as polyethylene terephthalate (PET), or similar polar semi-crystalline polymer. The use of a polar semi-crystalline polymer for the release liner 525 may substantially preclude wrinkling or other deformation of the dressing 110. For example, the polar semi-crystalline polymer may be highly orientated and resistant to softening, swelling, or other deformation that may occur when brought into contact with components of the dressing 110, or when subjected to temperature or environmental variations, or sterilization. Further, a release agent may be disposed on a side of the release liner 525 that is configured to contact the third layer 505. For example, the release agent may be a silicone coating and may have a release factor suitable to facilitate removal of the release liner 525 by hand and without damaging or deforming the dressing 110. In some embodiments, the release agent may be a fluorocarbon or a fluorosilicone, for example. In other embodiments, the release liner 525 may be uncoated or otherwise used without a release agent.

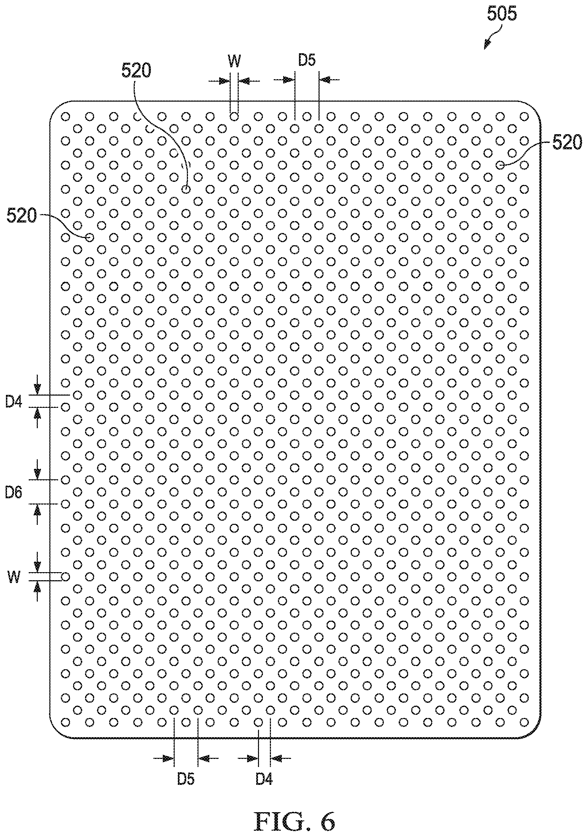

Figure 6 is a schematic view of an example configuration of the apertures 520, illustrating additional details that may be associated with some embodiments of the third layer 505. In some embodiments, the apertures 520 illustrated in Figure 6 may be associated only with an interior portion of the third layer 505. In the example of Figure 6, the apertures 520 are generally circular and have a width W, which may be about 2 millimeters in some examples. Figure 6 also illustrates an example of a uniform distribution pattern of the apertures 520. In Figure 6, the apertures 520 are distributed across the third layer 505 in a grid of parallel rows and columns. Within each row and column, the apertures 520 may be equidistant from each other, as illustrated in the example of Figure 6. The rows may be spaced a distance D4, and the apertures 520 within each of the rows may be spaced a distance D5. For example, a distance D4 of about 3 millimeters on center and a distance D5 of about 3 millimeters on center may be suitable for some embodiments. The apertures 520 in adjacent rows may be aligned or offset. For example, adjacent rows may be offset, as illustrated in Figure 6, so that the apertures are aligned in alternating rows separated by a distance D6. A distance D6 of about 6 millimeters may be suitable for some examples. The spacing of the apertures 520 may vary in some embodiments to increase the density of the apertures 520 according to therapeutic requirements.

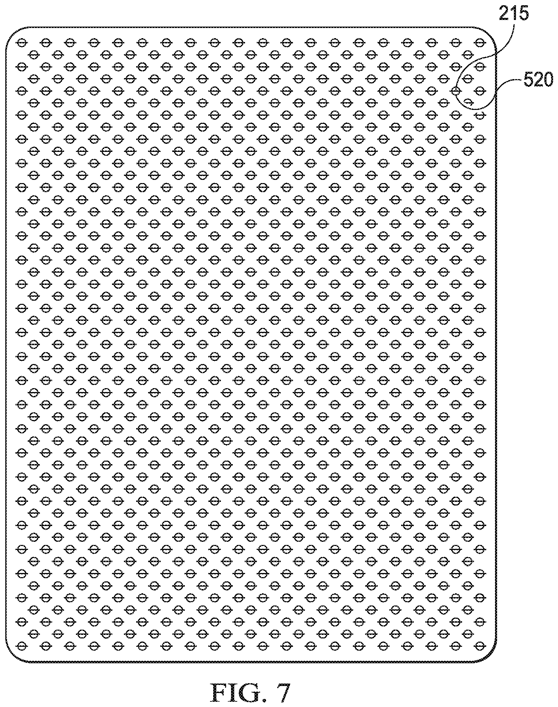

Figure 7 is a schematic view of the third layer 505 of Figure 6 overlaid on the first layer 205 of Figure 3, illustrating additional details that may be associated with some example embodiments of the tissue interface 120. For example, as illustrated in Figure 7, the perforations 215 may be aligned, overlapping, in registration with, or otherwise fluidly coupled to the apertures 520 in some embodiments. In some embodiments, one or more of the perforations 215 may be registered with the apertures 520 only in an interior portion, or only partially registered with the apertures 520. The perforations 215 in the example of Figure 7 are generally configured so that each of the perforations 215 is registered with only one of the apertures 520. In other examples, one or more of the perforations 215 may be registered with more than one of the apertures 520. For example, any one or more of the perforations 215 may extend across two or more of the apertures 520. Additionally or alternatively, one or more of the perforations 215 may not be registered with any of the apertures 520.

As illustrated in the example of Figure 7, the apertures 520 may be sized to expose a portion of the first layer 205, the perforations 215, or both through the third layer 505. In some embodiments, one or more of the apertures 520 may be sized to expose more than one of the perforations 215. For example, some or all of the apertures 520 may be sized to expose two or three of the perforations 215. In some examples, the length of each of the perforations 215 may be substantially equal to the diameter of each of the apertures 520. More generally, the average dimensions of the perforations are substantially similar to the average dimensions of the apertures 520. For example, the apertures 520 may be elliptical in some embodiments, and each of the perforations 215 may have a length L that is substantially equal to the major axis or the minor axis of the ellipse. In some embodiments, the dimensions of the perforations 215 may exceed the dimensions of the apertures 520, and the size of the apertures 520 may limit the effective size of the perforations 215 exposed through the third layer 505.

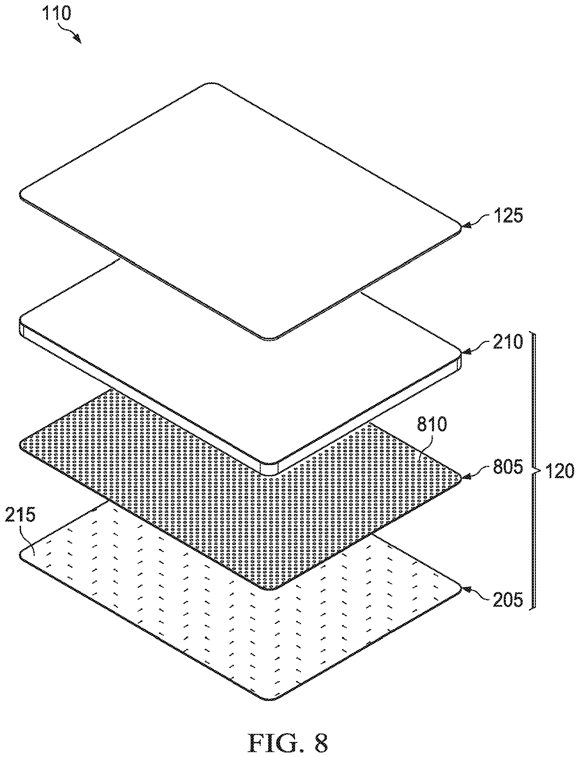

Figure 8 is an assembly view of another example of the dressing 110, illustrating additional details that may be associated with some example embodiments of the therapy system of Figure 1. In the example of Figure 8, the tissue interface 120 comprises a tie layer 805 in addition to the first layer 205 and the second layer 210. The tie layer 805 may have perforations 810 and may have a thickness between 10 microns and 100 microns in some embodiments. The tie layer 805 may be clear, colored, or printed. As illustrated in Figure 8, the tie layer 805 may be disposed between the first layer 205 and the second layer 210. The tie layer 805 may also be bonded to at least one of the first layer 205 and the second layer 210 in some embodiments.

The tie layer 805 may comprise polyurethane film, for example, which can be bonded to the first layer 205 and the second layer 210. For example, if the first layer 205 is formed of a polyethylene film and the second layer 210 is polyurethane foam, the first layer 205 may be more readily bonded to the tie layer 805 than directly to the second layer 210.

In some embodiments, one or more of the components of the dressing 110 may additionally be treated with an antimicrobial agent. For example, the second layer 210 may be a foam, mesh, or non-woven coated with an antimicrobial agent. In some embodiments, the second layer 210 may comprise antimicrobial elements, such as fibers coated with an antimicrobial agent. Additionally or alternatively, some embodiments of the first layer 205 may be a polymer coated or mixed with an antimicrobial agent. In other examples, the fluid conductor 230 may additionally or alternatively be treated with one or more antimicrobial agents. Suitable antimicrobial agents may include, for example, metallic silver, PHMB, iodine or its complexes and mixes such as povidone iodine, copper metal compounds, chlorhexidine, or some combination of these materials.

Additionally or alternatively, one or more of the components may be coated with a mixture that may include citric acid and collagen, which can reduce bio-films and infections. For example, the second layer 210 may be foam coated with such a mixture.

The cover 125, the first layer 205, the second layer 210, the third layer 505, or various combinations may be assembled before application or *in situ.* For example, the first layer 205 may be laminated to the second layer 210, and the cover 125 may be laminated to the second layer 210 opposite the first layer 205 in some embodiments. The third layer 505 may also be coupled to the first layer 205 opposite the second layer 210 in some embodiments. In some embodiments, one or more layers of the tissue interface 120 may coextensive. For example, the first layer 205 and the second layer 210 may be cut flush with the edge of the cover 125, exposing the edge of the second layer 210. In other embodiments, the first layer 205 may overlap the edge of the second layer 210.

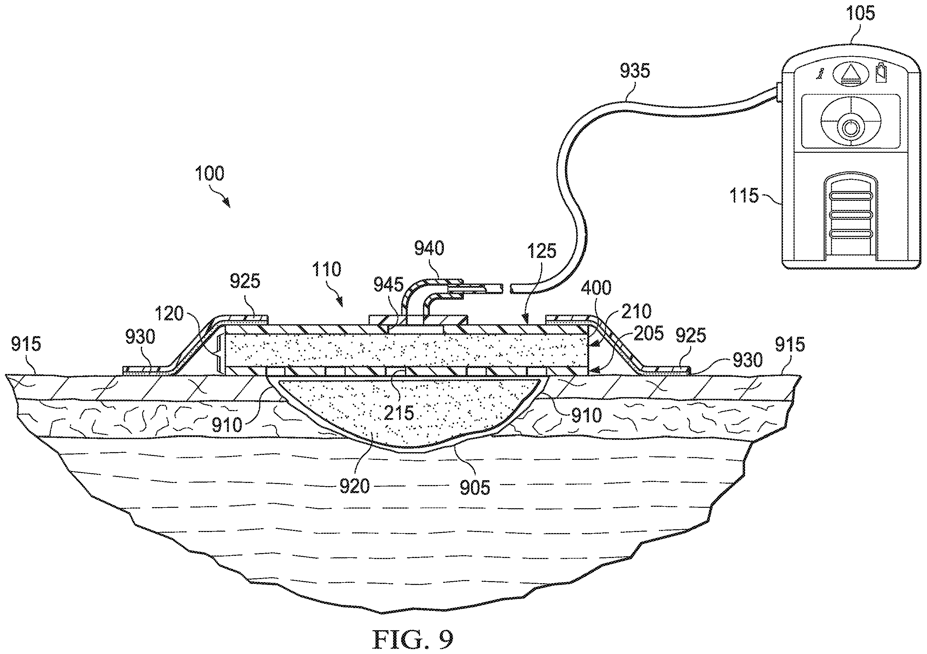

Figure 9 is a schematic diagram of an example of the therapy system 100 applied to a tissue site 905. In the example of Figure 9, the tissue site 905 is a surface wound. In use, a release liner (if included) may be removed to expose the tissue interface 120. The geometry and dimensions of the tissue interface 120, the cover 125, or both may vary to suit a particular application or anatomy. For example, the dressing 110 may be cut to size for a specific region or anatomical area, such as for amputations. The dressing 110 may be cut without losing pieces of the tissue interface 120 and without separation of the tissue interface 120.

The tissue interface 120 can be placed within, over, on, or otherwise proximate to the tissue site 905. In the example of Figure 9, the first layer 205 forms an outer surface of the dressing 110, and can be placed over the tissue site 905, including the edge 910 and epidermis 915. The first layer 205 may be interposed between the second layer 210 and the tissue site 905, which can prevent direct contact between the second layer 205 and epidermis 915. In other examples, the third layer 505 may form an outer surface of the dressing 110 and can provide temporary fixation over the tissue site 905.

As illustrated in the example of Figure 9, in some applications a filler 920 may also be disposed between the tissue site 905 and the first layer 205. For example, if the tissue site is a surface wound, the filler 920 may be applied interior to the edge 910, and the first layer 205 may be disposed over the filler 920. In some embodiments, the filler 920 may be a manifold, such as open-cell foam. The filler 920 may comprise or consist essentially of the same material as the second layer 210 in some embodiments.

In some examples, the dressing 110 may include one or more attachment devices 925. In some embodiments, one or more of the attachment devices 925 may comprise or consist essentially of a polymer strip, such as a polyurethane strip, having an adhesive 930 thereon. In some examples the adhesive 930 may be, for example, a medically-acceptable, pressure-sensitive adhesive that extends about a periphery, a portion, or an entire surface of each of the attachment devices 925. In some embodiments, for example, the adhesive 930 may be an acrylic adhesive having a coating weight between 25-65 grams per square meter (g.s.m.). Thicker adhesives, or combinations of adhesives, may be applied in some embodiments to improve the seal and reduce leaks. In some embodiments, such a layer of the adhesive 930 may be continuous or discontinuous. Discontinuities in the adhesive 930 may be provided by apertures or holes (not shown) in the adhesive 930. The apertures or holes in the adhesive 930 may be formed after application of the adhesive 930 or by coating the adhesive 930 in patterns on a carrier layer, such as, for example, a side of the attachment devices 925. Apertures or holes in the adhesive 930 may also be sized to enhance the MVTR of the attachment devices 925 in some example embodiments. In some embodiments, one or more of the attachment devices 925 may comprise or consist essentially of a composite strip of a perforated gel, substantially similar to the third layer 505, and a backing with an adhesive.

The attachment devices 925 can be disposed around edges of the cover 125, and the adhesive may pressed onto the cover 125 and epidermis 915 (or other attachment surface) to fix the dressing 110 in position and to seal the exposed perimeter 400 of the second layer 210.

Figure 9 also illustrates one example of a fluid conductor 935 and a dressing interface 940. As shown in the example of Figure 9, the fluid conductor 935 may be a flexible tube, which can be fluidly coupled on one end to the dressing interface 940. The dressing interface 935 may be an elbow connector, as shown in the example of Figure 9. In some examples, the tissue interface 120 can be applied to the tissue site 905 before the cover 125 is applied over the tissue interface 120. The cover 125 may include an aperture 945, or the aperture 945 may be cut into the cover 125 before or after positioning the cover 125 over the tissue interface 120. The aperture 945 of Figure 9 is centrally disposed. In other examples, the position of the aperture 945 may be off-center or adjacent to an end or edge of the cover 125. The dressing interface 935 can be placed over the aperture 945 to provide a fluid path between the fluid conductor 935 and the tissue interface 120. In other examples, the fluid conductor 935 may be inserted directly through the cover 125 into the tissue interface 120.

If not already configured, the dressing interface 940 may be disposed over the aperture 945 and attached to the cover 125. The fluid conductor 935 may be fluidly coupled to the dressing interface 940 and to the negative-pressure source 105.

Negative pressure from the negative-pressure source 105 can be distributed through the fluid conductor 925 and the dressing interface 930 to the tissue interface 120. Negative pressure applied through the tissue interface 120 can also create a negative pressure differential across the perforations 215 in the first layer 205, which can open or expand the perforations 215. For example, in some embodiments in which the perforations 215 may comprise substantially closed fenestrations through the first layer 205, a pressure gradient across the fenestrations can strain the adjacent material of the first layer 205 and increase the dimensions of the fenestrations to allow liquid movement through them, similar to the operation of a duckbill valve. Opening the perforations can allow exudate and other liquid movement through the perforations into the second layer 210. The second layer 210 can provide passage of negative pressure and exudate, which can be collected in the container 115.

Changes in pressure can also cause the second layer 210 to expand and contract. The first layer 205 can protect the epidermis 915 from irritation that could be caused by expansion, contraction, or other movement of the second layer 210. The first layer 205 can also substantially reduce or prevent exposure of a tissue site to the second layer 210, which can inhibit growth of tissue into the second layer 210.

If the negative-pressure source 105 is removed or turned off, the pressure differential across the perforations 215 can dissipate, allowing the perforations 215 to close and prevent exudate or other liquid from returning to the tissue site 905 through the first layer 205.

Additionally, or alternatively, instillation solution or other fluid may be distributed to the dressing 110, which can increase the pressure in the tissue interface 120. The increased pressure in the tissue interface 120 can create a positive pressure differential across the perforations 215 in the first layer 205, which can open the perforations 215 to allow the instillation solution or other fluid to be distributed to the tissue site 905.

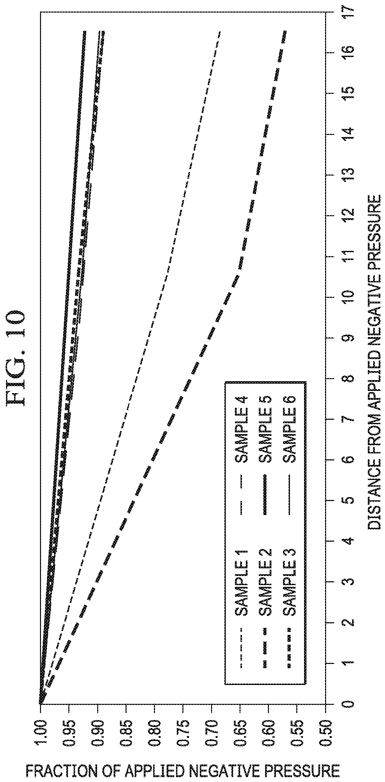

Figure 10 is a line chart that illustrates how the thickness T of a manifold, such as the second layer 210, can affect the manifolding performance in the presence of thick exudate. The data in the chart of Figure 10 reflects the change in negative pressure at increasing distances from a point at which a negative-pressure source is coupled to the manifold. More specifically, simulated wound fluid was instilled into various embodiments of the dressing 110 at the furthest point from the negative-pressure source. The simulated wound fluid was instilled at a rate of 500 cc/24 hours over a period of days, and the pressure in the manifold was monitored at known distances from the negative-pressure source over the instillation period and averaged. Sample 1 and Sample 2 each comprised a manifold having a thickness of about 6 millimeters; Sample 3 and Sample 4 each comprised a manifold having a thickness of about 8 millimeters; and Sample 5 and Sample 6 each comprised a manifold having a thickness of about 10 millimeters. The data illustrated in Figure 10 demonstrates that a manifold having a thickness of about 8 millimeters performs substantially better than configurations having a thickness of about 6 millimeters. More particularly, the negative pressure in Sample 1 and Sample 2 drops below 80% of the applied negative pressure at a distance of between 6 centimeters and 10 centimeters, while Sample 3 and Sample 4 maintain levels above 80% to distances greater than 16 centimeters. Significantly, Sample 3 and Sample 4 maintain an applied negative pressure comparably well to the configurations of Sample 5 and Sample 6 having a thickness of about 10 millimeters.

As Figure 10 illustrates, a manifold having a thickness of about 7 millimeters to about 9 millimeters may be advantageous for maintaining at least 80% of applied negative pressure larger dressings in the presence of viscous exudate. For example, if the dressing interface 940 is centrally disposed, as illustrated in Figure 9, a thickness of about 8 millimeters may be advantageous for manifolds having a length of at least 12 centimeters. In some embodiments, a thickness of about 8 millimeters may be particularly advantageous for manifolds having a length of at least 16 centimeters, up to a length of about 32 centimeters. If the dressing interface 940 is disposed toward an edge of the dressing, a thickness of 8 millimeters may be advantageous for manifolds having a length of about one-half of the length of a dressing having a centrally-disposed interface.

The systems, apparatuses, and methods described herein may provide significant advantages over prior dressings. For example, some dressings for negative-pressure therapy can require time and skill to be properly sized and applied to achieve a good fit and seal. In contrast, some embodiments of the dressing 110 can be simple to apply, reducing the time to apply and remove. In some embodiments, for example, the dressing 110 may be a fully-integrated negative-pressure therapy dressing that can be applied to a tissue site (including on the periwound) in one step, without being cut to size, while still providing or improving many benefits of other negative-pressure therapy dressings that require sizing. Some embodiments of the dressing 110 may alternatively be cut to size and readily sealed to a tissue site while still providing such benefits. Such benefits may include good manifolding, beneficial granulation, protection of the peripheral tissue from maceration, protection of the tissue site from shedding materials, and a low-trauma and high-seal bond. These characteristics may be particularly advantageous for surface wounds having moderate depth and medium-to-high levels of exudate. Some embodiments of the dressing 110 may remain on the tissue site for at least 5 days, and some embodiments may remain for at least 7 days. Antimicrobial agents in the dressing 110 may extend the usable life of the dressing 110 by reducing or eliminating infection risks that may be associated with extended use, particularly use with infected or highly exuding wounds.

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing 110, the container 115, or both may be separated from other components for manufacture or sale. In other example configurations, the controller 130 may also be manufactured, configured, assembled, or sold independently of other components.

The appended claims set forth novel and inventive aspects of the subject matter described above. Features, elements, and aspects described in the context of some embodiments may also be omitted, combined, or replaced by alternative features serving the same, equivalent, or similar purpose without departing from the scope of the invention defined by the appended claims.

## Claims

1. A dressing (110) for treating a tissue site (905) with negative pressure, comprising:
a first layer (205) comprising a polymer film having a plurality of perforations (215);
a second layer (210) comprising a manifold disposed adjacent to the polymer film, the manifold having a length of between 12 centimeters and about 32 centimeters and thickness of between 7 millimeters and about 9 millimeters; and
a cover (125) adjacent to the second layer (210), the cover (125) comprising a polymer film,
wherein the first layer (205), the second layer (210), and the cover (125) are stacked so that the second layer (210) is disposed between the first layer (205) and the cover (125).

2. The dressing (110) of claim 1, further comprising a dressing interface configured to be fluidly coupled to the manifold through the cover (125), optionally wherein the dressing interface is disposed at least 6 centimeters from an edge of the manifold, optionally wherein the dressing interface is disposed at least 12 centimeters from an edge of the manifold.

3. The dressing (110) of any of preceding claim, wherein the manifold comprises a foam having open cells.

4. The dressing (110) of claim 3, wherein the foam has a free volume of at least 90%.

5. The dressing (110) of claim 3, wherein the open cells have an average width in a range of about 400 microns to about 600 microns.

6. The dressing (110) of any of claims 3-5, wherein the open cells are reticulated.

7. The dressing (110) of any of claims 3-6, wherein the foam is polyurethane foam.

8. The dressing (110) of any of claims 3-7, wherein the foam is a polyurethane ether foam.

9. The dressing (110) of any preceding claim, wherein the second layer (210) has a perimeter that is exposed between the first layer (205) and the cover (125).

10. The dressing (110) of any preceding claim, wherein the perforations (215) comprise a plurality of slots, each of the slots having a length less than 5 millimeters.

11. The dressing (110) of any preceding claim, wherein the perforations (215) comprise a plurality of slots, each of the slots having a length less than 5 millimeters and a width less than 2 millimeters.

12. The dressing (110) of any preceding claim, wherein the perforations (215) comprise a plurality of slots, each of the slots having a length of about 2 millimeters to about 5 millimeters and a width of about 0.5 millimeters to about 2 millimeters.

13. The dressing (110) of any preceding claim, wherein the perforations (215) comprise a plurality of slits, each of the slits having a length less than 5 millimeters.

14. The dressing (110) of any preceding claim, wherein the perforations (215) comprise a plurality of slits, each of the slits having a length of about 2 millimeters to about 5 millimeters.

## Patentansprüche

1. Ein Verband (110) zum Behandeln einer Gewebestelle (905) mit Unterdruck, aufweisend:
eine erste Schicht (205), aufweisend eine Polymerfolie, die eine Vielzahl von Perforationen (215) aufweist;
eine zweite Schicht (210), aufweisend einen Verteiler, der benachbart zu der Polymerfolie angeordnet ist, wobei der Verteiler eine Länge von zwischen 12 Zentimeter und etwa 32 Zentimeter und eine Dicke von zwischen 7 Millimeter und etwa 9 Millimeter aufweist; und
eine Abdeckung (125), benachbart zu der zweiten Schicht (210), die Abdeckung (125) aufweisend eine Polymerfolie,
wobei die erste Schicht (205), die zweite Schicht (210) und die Abdeckung (125) gestapelt sind, sodass die zweite Schicht (210) zwischen der ersten Schicht (205) und der Abdeckung (125) angeordnet ist.

2. Der Verband (110) nach Anspruch 1, ferner aufweisend eine Verbandschnittstelle, die konfiguriert ist, um mit dem Verteiler durch die Abdeckung (125) fluidisch gekoppelt zu werden, optional wobei die Verbandschnittstelle mindestens 6 Zentimeter von einem Rand des Verteilers angeordnet ist, optional wobei die Verbandschnittstelle mindestens 12 Zentimeter von einem Rand des Verteilers angeordnet ist.

3. Der Verband (110) nach einem der vorstehenden Ansprüche, wobei der Verteiler einen Schaumstoff aufweist, der offene Zellen aufweist.

4. Der Verband (110) nach Anspruch 3, wobei der Schaumstoff ein freies Volumen von mindestens 90 % aufweist.

5. Der Verband (110) nach Anspruch 3, wobei die offenen Zellen eine durchschnittliche Breite in einem Bereich von etwa 400 Mikrometer bis etwa 600 Mikrometer aufweisen.

6. Der Verband (110) nach einem der Ansprüche 3 bis 5, wobei die offenen Zellen netzförmig sind.

7. Der Verband (110) nach einem der Ansprüche 3 bis 6; wobei der Schaumstoff Polyurethanschaumstoff ist.

8. Der Verband (110) nach einem der Ansprüche 3 bis 7, wobei der Schaumstoff ein Polyurethanetherschaumstoff ist.

9. Der Verband (110) nach einem der vorstehenden Ansprüche, wobei die zweite Schicht (210) einen Umfang aufweist, der zwischen der ersten Schicht (205) und der Abdeckung (125) freiliegt.

10. Der Verband (110) nach einem der vorstehenden Ansprüche, wobei die Perforationen (215) eine Vielzahl von Schlitzen aufweisen, wobei jeder der Schlitze eine Länge von weniger als 5 Millimeter aufweist.

11. Der Verband (110) nach einem der vorstehenden Ansprüche, wobei die Perforationen (215) eine Vielzahl von Schlitzen aufweisen, wobei jeder der Schlitze eine Länge von weniger als 5 Millimetern und eine Breite von weniger als 2 Millimetern aufweist.

12. Der Verband (110) gemäß einem der vorstehenden Ansprüche, wobei die Perforationen (215) eine Vielzahl von Schlitzen aufweisen, wobei jeder der Schlitze eine Länge von etwa 2 Millimeter bis etwa 5 Millimeter und eine Breite von etwa 0,5 Millimeter bis etwa 2 Millimetern aufweist.

13. Der Verband (110) nach einem der vorstehenden Ansprüche, wobei die Perforationen (215) eine Vielzahl von Schlitzen aufweisen, wobei jeder der Schlitze eine Länge von weniger als 5 Millimeter aufweist.

14. Der Verband (110) nach einem der vorstehenden Ansprüche, wobei die Perforationen (215) eine Vielzahl von Schlitzen aufweisen, wobei jeder der Schlitze eine Länge von etwa 2 Millimeter bis etwa 5 Millimetern aufweist.

## Revendications

1. Pansement (110) permettant de traiter un site tissulaire (905) par pression négative, comprenant :
une première couche (205) comprenant un film polymère ayant une pluralité de perforations (215) ;
une seconde couche (210) comprenant un collecteur disposé adjacent au film polymère, le collecteur ayant une longueur comprise entre 12 centimètres et environ 32 centimètres et une épaisseur comprise entre 7 millimètres et environ 9 millimètres ; et
une couverture (125) adjacente à la seconde couche (210), la couverture (125) comprenant un film polymère,
dans lequel la première couche (205), la seconde couche (210) et la couverture (125) sont empilées de sorte que la seconde couche (210) est disposée entre la première couche (205) et la couverture (125).

2. Pansement (110) selon la revendication 1, comprenant en outre une interface de pansement conçue pour être accouplée fluidiquement au collecteur à travers la couverture (125), éventuellement dans lequel l'interface de pansement est disposée à au moins 6 centimètres d'un bord du collecteur, éventuellement dans lequel l'interface de pansement est disposée à au moins 12 centimètres d'un bord du collecteur.

3. Pansement (110) selon l'une quelconque revendication précédente, dans lequel le collecteur comprend une mousse ayant des cellules ouvertes.

4. Pansement (110) selon la revendication 3, dans lequel la mousse a un volume libre d'au moins 90 %.

5. Pansement (110) selon la revendication 3, dans lequel les cellules ouvertes ont une largeur moyenne dans une plage comprise entre environ 400 microns et environ 600 microns.

6. Pansement (110) selon l'une quelconque des revendications 3-5, dans lequel les cellules ouvertes sont réticulées.

7. Pansement (110) selon l'une quelconque des revendications 3-6, dans lequel la mousse est une mousse de polyuréthane.

8. Pansement (110) selon l'une quelconque des revendications 3-7, dans lequel la mousse est une mousse d'éther de polyuréthane.

9. Pansement (110) selon l'une quelconque revendication précédente, dans lequel la seconde couche (210) a un périmètre qui est exposé entre la première couche (205) et la couverture (125).

10. Pansement (110) selon l'une quelconque revendication précédente, dans lequel les perforations (215) comprennent une pluralité de fentes, chacune des fentes ayant une longueur inférieure à 5 millimètres.

11. Pansement (110) selon l'une quelconque revendication précédente, dans lequel les perforations (215) comprennent une pluralité de fentes, chacune des fentes ayant une longueur inférieure à 5 millimètres et une largeur inférieure à 2 millimètres.

12. Pansement (110) selon l'une quelconque revendication précédente, dans lequel les perforations (215) comprennent une pluralité de fentes, chacune des fentes ayant une longueur d'environ 2 millimètres à environ 5 millimètres et une largeur d'environ 0,5 millimètre à environ 2 millimètres.

13. Pansement (110) selon l'une quelconque revendication précédente, dans lequel les perforations (215) comprennent une pluralité d'entailles, chacune des entailles ayant une longueur inférieure à 5 millimètres.

14. Pansement (110) selon l'une quelconque revendication précédente, dans lequel les perforations (215) comprennent une pluralité d'entailles, chacune des entailles ayant une longueur d'environ 2 millimètres à environ 5 millimètres.
